(19) <img>Europäisches Patentamt / European Patent Office / Office européen des brevets</img>

(11) **EP 3 596 476 B1**

(12) # EUROPEAN PATENT SPECIFICATION

(45) Date of publication and mention of the grant of the patent:
**10.01.2024   Bulletin 2024/02**

(21) Application number: **18714723.6**

(22) Date of filing: **14.03.2018**

(51) International Patent Classification (IPC):
***G01N 33/68*** *(2006.01)*

(52) Cooperative Patent Classification (CPC):
**G01N 33/6893;** G01N 2800/245; G01N 2800/50

(86) International application number:
**PCT/EP2018/056414**

(87) International publication number:
**WO 2018/167157 (20.09.2018 Gazette 2018/38)**

(54) **USE OF A MARKER SET FOR DETERMINING THE RISK OF KIDNEY REJECTION**

VERWENDUNG EINES MARKERSATZES ZUR BESTIMMUNG DES RISIKOS DER NIERENABSTOSSUNG

UTILISATION D'UN ENSEMBLE DE MARQUEURS POUR DÉTERMINER LE RISQUE DE REJET DE REIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **15.03.2017   DE 102017105566**

(43) Date of publication of application:
**22.01.2020   Bulletin 2020/04**

(73) Proprietor: **Numares AG**
**93053 Regensburg (DE)**

(72) Inventors:
• **EIGLSPERGER, Johannes**
  **84069 Walkenstetten (DE)**
• **NEUMANN, Sindy**
  **93047 Regensburg (DE)**
• **PAGEL, Philipp**
  **93105 Tegernheim (DE)**

• **ZUCKER, Maximilian**
  **80802 München (DE)**
• **PFAHLERT, Volker**
  **79400 Kandern (DE)**

(74) Representative: **Maikowski & Ninnemann Patentanwälte Partnerschaft mbB Postfach 15 09 20 10671 Berlin (DE)**

(56) References cited:
**WO-A1-2012/045773     US-A1- 2008 161 228**

• **K. SUHRE ET AL: "Urine Metabolite Profiles Predictive of Human Kidney Allograft Status", JOURNAL OF THE AMERICAN SOCIETY OF NEPHROLOGY., vol. 27, no. 2, 5 June 2015 (2015-06-05), pages 626-636, XP055472615, US ISSN: 1046-6673, DOI: 10.1681/ASN.2015010107**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The instant invention relates to the in vitro use of a marker set for determining the risk of kidney rejection according to the preamble of claim 1, to the in vivo diagnostic use of the same marker set according to the preamble of claim 9 and to a method for analyzing an isolated urine sample in vitro according to the preamble of claim 10.

It is of high medical interest to determine the risk of kidney rejection after kidney transplantation by an easily applicable method. For this purpose, WO 2012/045773 A9 discloses certain substances that are well suited for determining the according risk of kidney rejection, such as methyl malonate, lactate, methyl succinate, p-cresol, 3-hydroxy isovalerate, citrate, methyl guanidine, malonate, taurine, methyl guanidine, phenyl acetyl glycine, trigonelline, α-glucose, acetyl carnitine, phenyl acetate, and hippurate.

Also US 9,541,620 B2 discloses certain substances that might be used for determining the risk of kidney rejection. Thereby, it focuses on trimethylamine-N-oxide (TMAO) as most potent biomarker.

WO 2002/010755 A2 describes that increased levels of creatinine or urea in the blood serum are an indication for a failure of a kidney transplant.

Malyszko et al. describe in a publication that serum creatinine level - though connected to significant disadvantages - still remains the "gold standard" in assessment of kidney function (Malyszko, Jolanta, et al. "Biomarkers of delayed graft function as a form of acute kidney injury in kidney transplantation." Scientific Reports 5 (2015): 11684).

Suhre, K. et al. (2016) "Urine metabolite profiles predictive of human kidney allograft status", Journal of the American Society of Nephrology, 27(2), 626-636, describes urine metabolites indicative for the risk of rejection of a kidney transplant.

US 2008/0161228 A1 describes methods of identifying biochemical pathways. This patent application lists a wealth of small molecules that may be found in the urine of a subject.

It is an object of the instant invention to provide additional marker substances and appropriate marker combinations suited to determine the risk of kidney rejection and to enhance the overall performance of an according analysis method.

This object is achieved by the use of a novel marker set as defined in claim 1, i.e., in an in vitro method for determining (predicting) the risk of kidney rejection of an individual after having received a kidney donation. This determining is done by urine analysis. While according to prior art techniques generally individual substances are used as marker, the inventors found out that a sufficiently high accuracy of an according analysis method can only be achieved if at least three substances are used as markers. Thus, the instantly claimed use of a marker set requires the presence of at least three substances to be included in the marker set. These substances are to be chosen from the group consisting of 3-hydroxy isovalerate, acetyl carnitine, alanine, citrate, dimethylamine, glucose, glucuronate, hippurate, lactate, malonate, methyl guanidine, methyl malonate, methyl succinate, p-cresol, phenyl acetate, phenylacetylglycine, phenylacetylglutamine, taurine, trigonelline, and urea.

Some of these substances are already disclosed as suited markers for determining the risk of kidney rejection after kidney transplantation in WO 2012/045773 A9, namely 3-hydroxy isovalerate, acetyl carnitine, citrate, glucose, hippurate, lactate, malonate, methyl guanidine, methyl malonate, methyl succinate, p-cresol, phenyl acetate, phenylacetylglycine, taurine, and trigonelline. The inventors surprisingly found out that the accuracy of an according analysis method can be improved if alanine is necessarily present in the marker set. Therefore, the marker set, the use of which is instantly claimed, necessarily comprises alanine. Alanine, dimethylamine, glucuronate, phenylacetylglutamine, and urea will also referred to in the following as newly identified marker substances. The higher performance and thus better suitability of the marker set comprising at least one of these newly identified marker substances, in particular alanine, will be explained in more detail with respect to the exemplary embodiments.

It is possible that only alanine, i.e., one of the newly identified marker substances is present in the marker set. It is likewise possible that two, three, four, five, or six newly identified marker substances are present in the marker set, wherein alanine is always present. It is also possible that the marker set exclusively comprises newly identified marker substances, wherein the marker set comprises always alanine.

The individual substances being present in the marker set are used such that the concentration of these substances in a sample is determined. Then, the substance concentration can be used to calculate a score. The score is indicative for the risk of kidney rejection of an individual who previously underwent a kidney transplantation, i.e. who previously received a kidney donation.

In an embodiment, it is possible to define certain groups of risk for rejecting a kidney after kidney transplantation. Thus, it is possible to define a group of high-risk, a group of intermediate risk, a group of low risk and a group of very low risk in order to classify the determined or predicted risk of kidney rejection in an easily accessible way. It is also possible to use only some of these groups (e.g., high risk, intermediate risk, low risk; or high risk, low risk, very low risk) or to define additional groups.

When defining these groups (in particular on the basis of training data set) for a specific model making use of certain marker substances, it is suited to define the high-risk group such that at least 40%, in particular at least 45%, in particular at least 50% of the samples assigned to this group are samples from individuals that showed a kidney rejection. In an embodiment, the group of intermediate risk is defined such that 8 to 20%, in particular 10 to 17%, in particular 11 to 15% of all samples assigned to this group are samples from individuals that encountered a kidney rejection. In an embodiment, the group of low risk is defined such that 2 to 5%, in particular 2.5 to 4.5, in particular 3 to 4% of all samples assigned to this group are samples from individuals that encountered a kidney rejection. In an embodiment, the group of very low risk is defined such that not more than 1.5%, in particular not more than 1%, in particular not more than 0.5% of all samples assigned to this group are samples from individuals that encountered a kidney rejection. By such a grouping, a calculated score can be easily assigned to one of these groups so that the medical relevance of the calculated score is easily accessible.

In an embodiment, the marker set comprises 1, 2, 3, 4, 5, 6, 7, 8, 9, 10, 11, 12, 13, 14, 15, 16, 17, 18, 19, 20 or 21 individual substances. As already explained, 1, 2, 3, 4, 5 or 6 substances of the group consisting of alanine, dimethylamine, glucuronate, phenylacetylglutamine, and urea can be present in the marker set, wherein alanine is necessarily present in the marker set.

In an embodiment, the marker set necessarily comprises at least one substance chosen from the subgroup of newly identified marker substances consisting of dimethylamine, glucuronate, and urea. Besides alanine, these newly identified marker substances have been proven to be especially suited for enhancing the overall performance of the marker set for determining the risk of kidney rejection.

In another embodiment, the marker set necessarily comprises urea. Urea turned out to be one of the most suited substances to increase the overall performance of the marker set.

In a further embodiment, the marker set comprises necessarily urea and alanine. In a further embodiment, the marker set necessarily comprises urea, alanine and glucuronate. In a further embodiment, the marker set comprises necessarily urea, alanine, and dimethylamine.

In an embodiment, the marker set comprises lactate and citrate as already principally known suited marker substances in combination with alanine.

In an embodiment, the marker set does not comprise dimethylamine, lactate, acetate and alanine at the same time.

In an embodiment, the marker set does not comprise only dimethylamine, lactate and alanine.

In an embodiment, the marker set does not comprise only dimethylamine, acetate and alanine.

In an embodiment, the marker set does not comprise only lactate, acetate and alanine.

The following examples of marker sets are particularly suited marker sets:

- Lactate, citrate, urea and alanine.
- Lactate, citrate, urea, alanine and glucuronate.
- Lactate, citrate, urea, alanine and dimethylamine.

The instant invention relates in an aspect also to the use of the same marker set for in vivo diagnostics of the risk of kidney rejection of an individual after having received a transplanted kidney. Thus, in this aspect, the invention relates to a marker set comprising at least three substances chosen from the group consisting of 3-hydroxy isovalerate, acetyl carnitine, alanine, citrate, dimethylamine, glucose, glucuronate, hippurate, lactate, malonate, methyl guanidine, methyl malonate, methyl succinate, p-cresol, phenyl acetate, phenylacetylglycine, phenylacetylglutamine, taurine, trigonelline, and urea, for use in in-vivo diagnostics by urine analysis of the risk of kidney rejection of an individual after having received a kidney donation, with the provision that the marker set comprises alanine.

Embodiments of the in vitro use of the marker set explained above are likewise applicable to an in vivo use or the diagnostic use of the marker set in any desired combination.

The claimed use of the marker set in in-vivo diagnostics constitutes another medical indication of this marker set. Thereby, alanine is always present in the marker set.

In an aspect, the invention also relates to method of analyzing an isolated urine sample in vitro. This method comprises the steps explained in the following and makes use of a urine sample from an individual who has received a kidney donation is provided. This urine sample can be taken at any time after kidney transplantation, e.g., 0 days to 400 days after transplantation, in particular 1 day to 365 days after transplantation, in particular 2 days to 350 days after transplantation, in particular 3 days to 340 days after transplantation, in particular 4 days to 330 days after transplantation, in particular 5 days to 320 days after transplantation, in particular 6 days to 310 days after transplantation, in particular 7 days to 300 days after transplantation, in particular 8 days to 290 days after transplantation, in particular 10 days to 280 days after transplantation, in particular 11 days to 270 days after transplantation, in particular 12 days to 260 days after transplantation, in particular 13 days to 250 days after transplantation, in particular 14 days to 240 days after transplantation, in particular 15 days to 230 days after transplantation, in particular 16 days to 220 days after transplantation, in particular 17 days to 210 days after transplantation, in particular 18 days to 200 days after transplantation, in particular 19 days to 190 days after transplantation, in particular 20 days to 180 days after transplantation, in particular 25 days to 170 days after transplantation, in particular 30 days to 160 days after transplantation, in particular 40 days to 150 days after transplantation, in particular 50 days to 140 days after transplantation, in particular 60 days to 130 days after transplantation, in particular 70 days to 120 days after transplantation, in particular 80 days to 110 days after transplantation, in particular 90 days to 100 days after transplantation. Thereby, "0 days" means less than 24 hours after kidney transplantation.

The concentration of at least three substances chosen from the group consisting of 3-hydroxy isovalerate, acetyl carnitine, alanine, citrate, dimethylamine, glucose, glucuronate, hippurate, lactate, malonate, methyl guanidine, methyl malonate, methyl succinate, p-cresol, phenyl acetate, phenylacetylglycine, phenylacetylglutamine, taurine, trigonelline, and urea, is determined with a suited measuring technique. These substances serve as marker substances. Thereby, the concentration of alanine is necessarily determined.

A very well suited measuring technique for determining the concentration of the individual substances is nuclear magnetic resonance spectroscopy (NMR spectroscopy).

The determined concentrations are afterwards used to calculate a score. Thereby, the score is indicative for the risk of kidney rejection of the individual.

It is generally possible to calculate the score in any desired way. A suited way to calculate the score is disclosed on pages 25 to 27 of WO 2012/045773 A9. Another suited way to calculate the score is the following:

$$score = \frac{1}{1+e^{-\omega}} \, ,$$

wherein

$$\omega = a + \sum_{x=1}^{n} b_x \cdot I_x$$

a = const.
$b_x$ = substance specific coefficient
I = parameter being indicative for the concentration of substance x

Thereby, the individual factors a, b need to be adjusted according to the underlying model and can vary in dependence on the specific substances considered in the marker set. Parameter "I" can be, e.g., the signal intensity of an according signal observed in the evaluated measuring result. To give an example, "I" can be the signal intensity of an NMR signal in an NMR spectrum if NMR spectroscopy is used as measuring technique.

In an embodiment, the calculated score is output and presented to the individual and/or to a third person such as a physician or medical staff. The output can be performed on a display (i.e., in an electronic way) or in printed form. Thereby, it is also possible to generate a report indicating the score, optionally in combination with a comparative scale of possible scores and their meaning with respect to the risk of kidney rejection after kidney transplantation.

In an embodiment, the sample is grouped into one of at least two predefined groups on the basis of the calculated score. The according grouping can also be indicated on an according report.

Using a urine sample is particularly suited for carrying out the method since urine samples are easily collectable from individuals and do not need any closer interaction with the body of the individual.

To compensate for differing overall marker concentrations in different samples, the determined concentrations are, in an embodiment, normalized to the additionally determined concentration of creatinine. If a person, from whom the analyzed urine sample has been obtained, has ingested a big volume of liquid, his or her total urine volume is also big. At the same time, the overall concentration of the marker substances (and other substances) in the urine is in such a case generally lower than in a sample taken from a person who has ingested a lower volume of liquid and has a lower total urine volume. If the determined marker concentrations are divided by the creatinine concentration in the same urine sample (i.e., normalized to the creatinine concentration), these effects are erased.

The score referred to above can form the basis of a diagnostic test being based on at least three metabolites.

In an embodiment, an extended score is calculated starting from the score by additionally considering at least one further value. This can further increase the performance of a diagnostic test being based on the instantly described method. This further value is chosen from the group consisting of a value being indicative for the concentration of creatinine in blood serum of the individual, a value being indicative for the glomerular filtration rate (GFR) of the individual, and a value being indicative for the estimated glomerular filtration rate (eGFR) of the individual. Thereby, it is particularly suited to calculate an extended score by considering a value being indicative for the concentration of creatinine in blood serum. In doing so, both information relating to the concentration of individual marker substances in the urine of the individual (patient) as well as information relating to the concentration of creatinine in the blood serum of the individual are taken into account. This additionally enhances the reliability of the whole method with respect to the correctness of the calculated extended score.

The combination of the score based on metabolites and additional information based on creatinine concentration in blood serum, GFR and/or eGFR results in a decision support system. Such a decision support system is particularly suited to group different samples into different groups (such as different risk groups for a specific disease or indication like the risk of kidney rejection after kidney transplantation of an individual who has received a kidney donation). Based on such a grouping, a physician can be assisted in assessing the medical relevance of the obtained result or in making a diagnosis.

The described in vitro method can also be regarded as a method for providing original data that can afterwards be used by a physician to make a diagnosis regarding the risk of kidney rejection after kidney transplantation. This diagnostic step does not form part of the claimed method.

Herewith disclosed is a decision support system implementing a method as explained above.

Herewith disclosed is the use of the creatinine concentration in a urine sample for normalizing the concentration of at least one other substance determined in the same urine sample, in particular by dividing the concentration of the other substance by the concentration of creatinine. This normalization method can, in an embodiment, combined with any other method or use described or claimed herein.

All explained embodiments of the described uses can be applied in any desired combination to the described methods, and vice versa.

Aspects of the invention will now explained in more detail with respect to exemplary embodiments and accompanying Figures. In the Figures:

Figure 1A    shows a receiver operating characteristic (ROC) plot of sensitivity versus specificity of a model for determining the risk of kidney rejection taking into account lactate, citrate and trigonelline as marker substances (comparative example);

Figure 1B    shows an ROC plot of sensitivity versus specificity of a model for determining the risk of kidney rejection taking into account lactate, citrate and hippurate as marker substances (comparative example);

Figure 1C    shows an ROC plot of sensitivity versus specificity of a model for determining the risk of kidney rejection taking into account lactate, citrate and urea as marker substances;

Figure 2A    shows an ROC plot of sensitivity versus specificity of a model for determining the risk of kidney rejection taking into account lactate, citrate, trigonelline and glucose as marker substances (comparative example);

Figure 2B    shows an ROC plot of sensitivity versus specificity of a model for determining the risk of kidney rejection taking into account lactate, citrate, hippurate and glucose as marker substances (comparative example);

Figure 2C    shows an ROC plot of sensitivity versus specificity of a model for determining the risk of kidney rejection taking into account lactate, citrate, trigonelline and hippurate as marker substances (comparative example);

Figure 2D    shows an ROC plot of sensitivity versus specificity of a model for determining the risk of kidney rejection taking into account lactate, citrate, urea and alanine as marker substances;

Figure 2E    shows an ROC plot of sensitivity versus specificity of a model for determining the risk of kidney rejection taking into account lactate, citrate, urea and glucuronate as marker substances;

Figure 2F    shows an ROC plot of sensitivity versus specificity of a model for determining the risk of kidney rejection taking into account lactate, citrate, urea and alanine as marker substances and additionally considering the concentration of creatinine in blood serum of the individual;

Figure 2G    shows an ROC plot of sensitivity versus specificity of a model for determining the risk of kidney rejection taking into account lactate, citrate, urea and alanine as marker substances and additionally considering the estimated glomerular filtration rate of the individual;

Figure 3A    shows an ROC plot of sensitivity versus specificity of a model for determining the risk of kidney rejection taking into account lactate, citrate, trigonelline, hippurate and glucose as marker substances (comparative example);

Figure 3B    shows an ROC plot of sensitivity versus specificity of a model for determining the risk of kidney rejection taking into account lactate, citrate, urea, alanine and glucuronate as marker substances; and

Figure 3C    shows an ROC plot of sensitivity versus specificity of a model for determining the risk of kidney rejection taking into account lactate, citrate, urea, alanine and dimethylamine as marker substances.

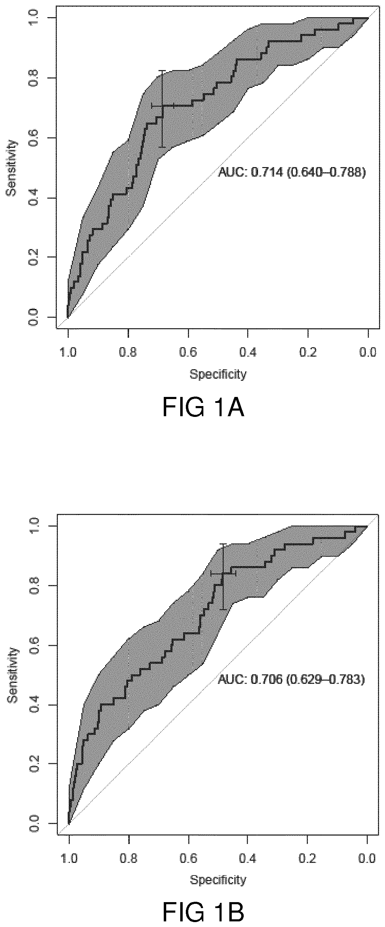

The results depicted in Figures 1A to 3C are based on an analysis of more than 4200 urine samples from patients having obtained a kidney donation. The urine samples were taken from these patients in a time window between 14 days after transplantation and 365 days after transplantation. Thereby, in a first step a training sample set (based on more than 870 samples) has been analyzed to identify suited marker substances. Subsequently, the identified suited marker substances have been tested on a validation sample set (more than 580 samples) to assess the performance of a determination of the risk of kidney rejection making use of these marker substances.

**Sample handling and preparation**

Mid-stream urine samples were collected in standard plastic urine cups and aliquots of 1 ml were transferred into 1.5 ml sample tubes. The aliquots were frozen at -20°C within 8 hours after collection. For storage, the tubes were kept at -80°C. For NMR measurements, aliquots were allowed to thaw at room temperature. Upon complete thawing, 600 $\mu$l of the aliquot were mixed with 120 $\mu$l of Axinon® urine additive solution. The mixture was centrifuged at 20000 g for 10 min and 600 $\mu$l of the supernatant was transferred to 5 mm NMR tubes and kept at 4-8°C until measurement.

**NMR measurements**

All measurements were carried out on a Bruker Avance II+ 600MHz NMR spectrometer using a PATXI 1 H/D-13C/15N Z-GRD probe. All samples were kept at 5-7°C in the SampleJet and brought to the target temperature in the integrated preheating block before measurement. A standard pulse program with 30 degree excitation pulse and pre-saturation for water suppression was used (zgpr30).

Samples were measured in batches of up to 93 samples per run. In addition to the analytical samples, each run included one Axinon® urine calibrator sample and two Axinon® urine control samples (before and after the analytical urine samples, respectively) in order to assure ideal measurement conditions throughout the run.

**Signal analysis**

NMR spectra underwent automatic referencing, phase correction and baseline correction before further analysis.

Subsequently, the NMR spectra underwent an automatic standardization and calibration procedure to minimize between-device, between-day and between-run effects. The quality of each of these spectra was assessed by a custom spectrum qualification algorithm that analyzes general spectral properties, e.g., offset and tilt of the baseline in selected spectral regions, and properties of selected indicator signals, e.g., signal position, shape and width. Spectra that did not meet the predefined quality criteria were excluded from further analysis. Successfully qualified spectra were divided into 403 bins of 0.04 ppm width positioned with 50% overlap between consecutive bins covering a spectral range from 0.96 to 9.04 ppm. The area from 4.5 to 5.0 ppm (water signal) was not considered in the analyses. Thus, a total of 377 bins for subsequent analysis remained.

Quantification of specific signal peaks was done by fitting Pseudo-Voigt functions, which represent a linear combination of a Gaussian and a Lorentzian function, to each peak of interest. The resulting signal fits were checked for goodness of fit in order to reject results of insufficient fit quality.

**Marker substance identification**

Suited marker substances were identified with the aid of the obtained NMR spectra in an iterative way by combining the NMR information with the additional information whether the underlying sample was taken from an individual that showed kidney rejection or from an individual that did not show kidney rejection.

An individual who recovered well from kidney transplantation and did not show any significant abnormality was considered to be an individual that did not show kidney rejection. Individuals encountering kidney problems after kidney transplantation were subjected to a biopsy being the gold standard for determining whether or not the donated kidney is rejected. If the outcome of such a biopsy was that kidney rejection has been taken place or was about to take place, these individuals were considered to be individuals showing kidney rejection.

Since a biopsy can cause hematuria and may potentially impact kidney function directly, only urine samples that have been taken within max. 7 days prior to biopsy were included in the assessment. Thus, if a urine sample was taken from an individual that subsequently underwent a biopsy which resulted in the finding that a kidney rejection was about to take place or has been taken place, the sample was assigned to an individual having a high risk of kidney rejection. If a biopsy did not provide any result indicative for kidney rejection, the according individual was considered to be a person having no significant risk of kidney rejection.

**Test of identified marker substances**

The identified marker substances were tested in different combinations to assess their suitability for determining the risk of kidney rejection of an individual after kidney transplantation. In doing so, the result of the determination based on the marker substances (predicted risk) has been checked against clinical signs of kidney rejection and/or transplant dysfunction as well as the results of performed biopsies (histologically proven kidney rejection).

The results are summarized in receiver operating characteristic (ROC) plots. In these plots, the area under the curve (AUC) indicates the fitness of the prediction. If the AUC is 0.5, the prediction is to be considered random and thus not well suited. The higher the AUC, the better is the prediction model.

Figure 1A shows an ROC plot of a model for determining the risk of kidney rejection taking into account lactate, citrate and trigonelline as marker substances (comparative example). These substances are already known from prior art to be used as substances for determining the risk of kidney rejection, but have not been described so far in this specific combination. To compensate for concentration differences, all substance concentrations determined in this and in the following examples have been normalized to the concentration of creatinine in urine. Thus, the concentration of creatinine in urine serves as internal standard. The resulting AUC is 0.714. This AUC is much better than a simply random prediction and also better than an according AUC of an individual substance used as marker for determining the risk of kidney rejection. Nonetheless, a further enhancement of the reliability of the prediction model is desired.

If trigonelline is replaced by hippurate, the AUC is 0.706 and thus even lower (comparative example) as can be seen from Figure 1B. Hippurate is also already known from prior art to be used as marker for determining the risk of kidney rejection.

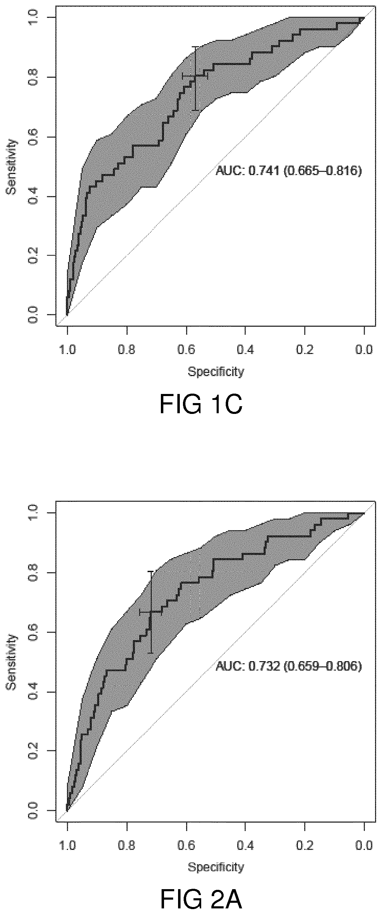

If, however, trigonelline is replaced by a newly identified marker substance, namely, urea, the AUC significantly increases to 0.741. This is shown in Figure 1C. Thus, a combination of two "old" marker substances (lactate and citrate) with a newly identified marker substance (urea) results in a much higher performance of the respective prediction model.

Generally, the quality of the prediction model can be increased by increasing the total number of marker substances included in the prediction model. Figure 2A shows an ROC plot of the model for determining the risk of kidney rejection taking into account lactate, citrate, trigonelline and glucose (comparative example). Using these four already known substances as marker substances, an AUC of 0.732 results.

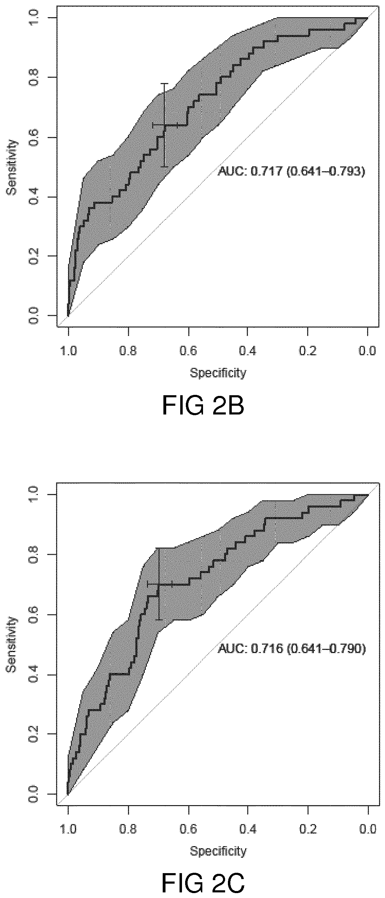

By using hippurate instead of trigonelline, the AUC drops to 0.717, as shown in Figure 2B (comparative example).

When using a combination of lactate, citrate, trigonelline and hippurate as marker substances in a model for determining the risk of kidney rejection, an AUC of 0.716 results, as shown in Figure 2C (comparative example).

Thus, a model making use of four already known marker substances results in an AUC ranging from 0.716 to 0.732.

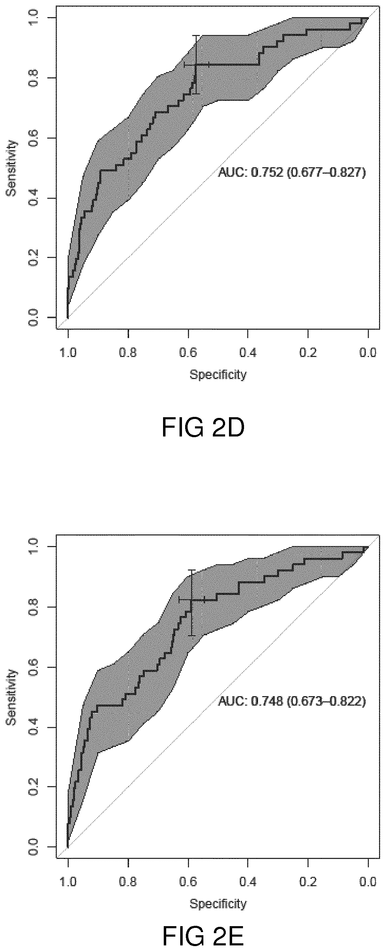

The performance of an according model for predicting the risk of kidney rejection can be significantly increased by including two newly identified marker substances into the model. If the model is based on lactate, citrate, urea and alanine, an AUC of 0.752 results, as shown in Figure 2D.

A similar high AUC can be observed for a model taking into account lactate, citrate, urea and glucuronate. This is shown in Figure 2E, indicating an AUC of 0.748.

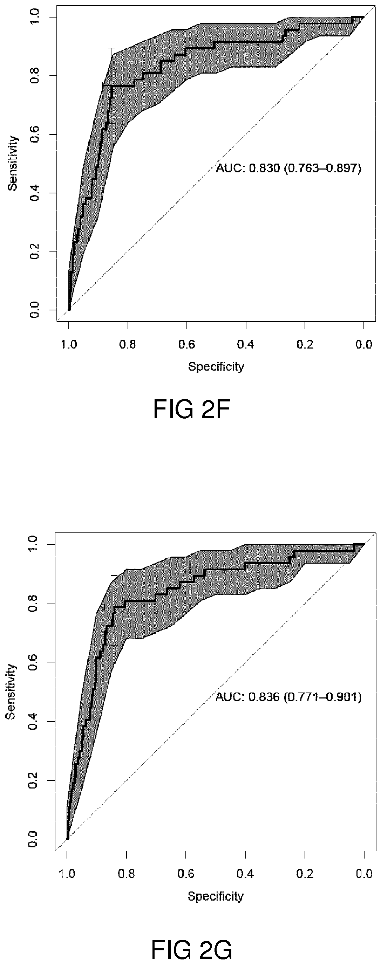

The prediction quality of an according model for determining the risk of kidney rejection after kidney transplantation can be even more increased if additional patient information is included in the model. Figure 2F shows an ROC plot of a model taking into account lactate, citrate, urea and alanine as marker substances (like the model underlying Figure 2D). By additionally considering the creatinine concentration in the blood serum of the same individual, the AUC can be increased from 0.752 (cf. Figure 2D) to 0.830, as shown in Figure 2F. Thereby, the calculated score for the predicted risk of kidney rejection has been multiplied by the concentration of creatinine in the blood serum of the same individual, a urine sample of whom has been analyzed.

A likewise high performance increase can be observed when combining a prediction model with additional information based on the estimated glomerular filtration rate (eGFR). Figure 2G shows the performance of a model taking into account lactate, citrate, urea and alanine as marker substances (cf. Figure 2D) and additionally considering the estimated glomerular filtration rate as further parameter. In doing so, the AUC increases from 0.752 (cf. Figure 2D) to 0.836, as indicated in Figure 2G. Thereby, the score for predicting the risk of kidney rejection calculated on the basis of lactate, citrate, urea and alanine has been divided by the determined estimated glomerular filtration rate of the same individual, a urine sample of whom has been analyzed.

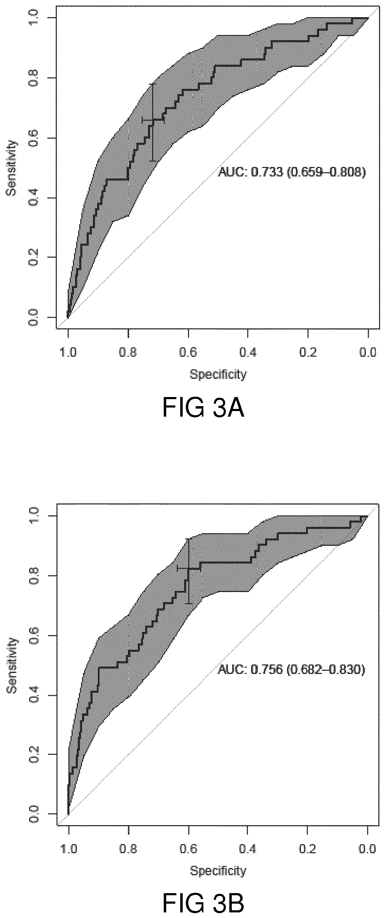

If five instead of four already known marker substances are used in a marker set of a model for predicting the risk of kidney rejection after kidney transplantation, the AUC can only hardly be increased. This is shown in Figure 3A depicting an ROC plot of a model taking into account lactate, citrate, trigonelline, hippurate and glucose as marker substances (comparative example). The resulting AUC is 0.733 and thus only slightly higher than the AUC of a model taking into account lactate, citrate, trigonelline and glucose as marker substances (cf. Figure 2A).

If, however, two already known marker substances (lactate and citrate) are combined with three newly identified substances, the prediction quality of the model can be significantly increased, as indicated by a significantly increased AUC. Specifically, if a model is used for predicting the risk of kidney rejection after kidney transplantation that takes into account lactate, citrate, urea, alanine and glucuronate as marker substances, an AUC of 0.756 results, as indicated in Figure 3B.

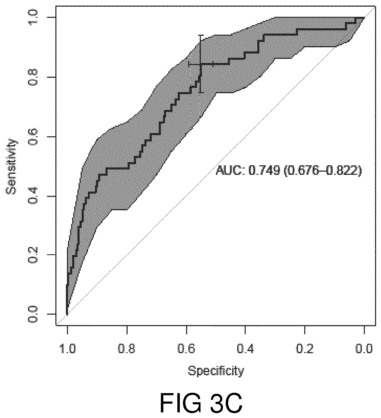

If glucuronate is replaced by dimethylamine, the resulting AUC is 0.749 and thus slightly lower, but still much higher than without these newly identified marker substances. An according ROC plot is shown in Figure 3C.

Summarizing, the newly identified marker substances alanine, dimethylamine, glucuronate, phenylacetylglutamine, and urea strongly increase the reliability and performance of prediction models for assessing the risk of kidney rejection after kidney transplantation if used alone or in combination with already known marker substances for this purpose. Thus, by the (additional) use of these marker substances, methods for determining or predicting the risk of kidney rejection after kidney transplantation can be performed with a much higher accuracy and reliability. This is likewise true for methods providing original data that can subsequently be used by a physician to make a diagnosis regarding the kidney rejection risk.

**Claims**

1.  Use of a marker set comprising at least three substances chosen from the group consisting of 3-hydroxy isovalerate, acetyl carnitine, alanine, citrate, dimethylamine, glucose, glucuronate, hippurate, lactate, malonate, methyl guanidine, methyl malonate, methyl succinate, p-cresol, phenyl acetate, phenylacetylglycine, phenylacetylglutamine, taurine, trigonelline, and urea, in an in vitro method for determining the risk of kidney rejection of an individual after having obtained a kidney donation by urine analysis, with the provision that the marker set comprises alanine.

2.  Use according to claim 1, **characterized in that** the marker set comprises at least one substance chosen from the group consisting of dimethylamine, glucuronate, and urea.

3.  Use according to claim 1, **characterized in that** the marker set comprises urea.

4.  Use according to any of the preceding claims, **characterized in that** the marker set comprises lactate and citrate.

5.  Use according to any of the preceding claims, **characterized in that** the marker set does not comprise dimethylamine, lactate, acetate and alanine at the same time.

6.  Use according to any of the preceding claims, **characterized in that** the marker set does not comprise only dimethylamine, lactate and alanine.

7.  Use according to any of the preceding claims, **characterized in that** the marker set does not comprise only dimethylamine, acetate and alanine.

8.  Use according to any of the preceding claims, **characterized in that** the marker set does not comprise only lactate, acetate and alanine.

9.  Marker set comprising at least three substances chosen from the group consisting of 3-hydroxy isovalerate, acetyl carnitine, alanine, citrate, dimethylamine, glucose, glucuronate, hippurate, lactate, malonate, methyl guanidine, methyl malonate, methyl succinate, p-cresol, phenyl acetate, phenylacetylglycine, phenylacetylglutamine, taurine, trigonelline, and urea, for use in in-vivo diagnostics of the risk of kidney rejection of an individual after having received a kidney donation by urine analysis, with the provision that the marker set comprises alanine.

10. Method for analyzing an isolated urine sample in vitro, comprising the following steps:

    a) determining the concentration of at least three substances chosen from the group consisting of 3-hydroxy isovalerate, acetyl carnitine, alanine, citrate, dimethylamine, glucose, glucuronate, hippurate, lactate, malonate, methyl guanidine, methyl malonate, methyl succinate, p-cresol, phenyl acetate, phenylacetylglycine, phenylacetylglutamine, taurine, trigonelline, and urea in a urine sample from an individual who has received a kidney donation by analyzing the urine sample with a suited measuring technique, with the provision that the concentration of alanine is determined, and
    b) calculating a score from the determined concentrations, the score being indicative for the risk of kidney rejection of the individual.

11. Method according to claim 10, **characterized in that** the determined concentrations are normalized to the concentration of creatinine in the same urine sample.

12. Method according to claim 10 or 11, **characterized in that** that the urine sample is grouped into one of at least two predefined groups on the basis of the calculated score.

13. Method according to claim 10 or 11, **characterized in that** an extended score is calculated from the score by additionally considering at least one value of the group consisting of a value being indicative for the concentration of creatinine in blood serum of the individual, a value being indicative for the glomerular filtration rate of the individual, and a value being indicative for the estimated glomerular filtration rate of the individual.

14. Method according to claim 13, **characterized in that** the sample is grouped into one of at least two predefined groups on the basis of the calculated extended score, thus providing a decision support system.

**Patentansprüche**

1.  Verwendung eines Markersatzes, umfassend zumindest drei Substanzen, ausgewählt aus der Gruppe bestehend aus 3-Hydroxyisovalerat, Acetylcarnitin, Alanin, Citrat, Dimethylamin, Glucose, Glucuronat, Hippurat, Lactat, Malonat, Methylguanidin, Methylmalonat, Methylsuccinat, p-Kresol, Phenylacetat, Phenylacetylglycin, Phenylacetylglutamin, Taurin, Trigonellin und Harnstoff, in einem In-vitro-Verfahren zur Bestimmung des Risikos einer Nierenabstoßung eines Individuums nach Erhalt einer Nierenspende durch Urinanalyse, mit der Maßgabe, dass der Markersatz Alanin aufweist.

2. Verwendung nach Anspruch 1, **dadurch gekennzeichnet, dass** der Markersatz zumindest eine Substanz umfasst, ausgewählt aus der Gruppe bestehend aus Dimethylamin, Glucuronat und Harnstoff.

3. Verwendung nach Anspruch 1, **dadurch gekennzeichnet, dass** der Markersatz Harnstoff umfasst.

4. Verwendung nach einem der vorhergehenden Ansprüche, **dadurch gekennzeichnet, dass** der Markersatz Lactat und Citrat umfasst.

5. Verwendung nach einem der vorhergehenden Ansprüche, **dadurch gekennzeichnet, dass** der Markersatz nicht zugleich Dimethylamin, Lactat, Acetat und Alanin umfasst.

6. Verwendung nach einem der vorhergehenden Ansprüche, **dadurch gekennzeichnet, dass** der Markersatz nicht alleinig Dimethylamin, Lactat und Acetat umfasst.

7. Verwendung nach einem der vorhergehenden Ansprüche, **dadurch gekennzeichnet, dass** der Markersatz nicht alleinig Dimethylamin, Acetat und Alanin enthält.

8. Verwendung nach einem der vorhergehenden Ansprüche, **dadurch gekennzeichnet, dass** der Markersatz nicht alleinig Lactat, Acetat und Alanin umfasst.

9. Markersatz umfassend zumindest drei Substanzen, ausgewählt aus der Gruppe bestehend aus 3-Hydroxyisovalerat, Acetylcarnitin, Alanin, Citrat, Dimethylamin, Glucose, Glucuronat, Hippurat, Lactat, Malonat, Methylguanidin, Methylmalonat, Methylsuccinat, p-Kresol, Phenylacetat, Phenylacetylglycin, Phenylacetylglutamin, Taurin, Trigonellin und Harnstoff, zur Verwendung in der In-vivo-Diagnostik des Risikos einer Nierenabstoßung eines Individuums nach Erhalt einer Nierenspende durch Urinanalyse, mit der Maßgabe, dass der Markersatz Alanin aufweist.

10. Verfahren zur In-vitro-Analyse einer isolierten Urinprobe, das die folgenden Schritte umfasst:

a) Ermitteln der Konzentration von zumindest drei Substanzen, ausgewählt aus der Gruppe bestehend aus 3-Hydroxyisovalerat, Acetylcarnitin, Alanin, Citrat, Dimethylamin, Glucose, Glucuronat, Hippurat, Lactat, Malonat, Methylguanidin, Methylmalonat, Methylsuccinat, p-Kresol, Phenylacetat, Phenylacetat, Phenylacetat, Phenylacetat, Phenylacetat, Phenylacetat, Phenylacetylglycin, Phenylacetylglutamin, Taurin, Trigonellin und Harnstoff in einer Urinprobe eines Individuums, das eine Nierenspende erhalten hat, durch Analyse der Urinprobe mit einer geeigneten Messtechnik, mit der Maßgabe, dass die Konzentration von Alanin ermittelt wird, und

b) Berechnen eines Kennwerts aus den ermittelten Konzentrationen, wobei der Kennwert ein Indikator für das Risiko einer Nierenabstoßung des Individuums ist.

11. Verfahren nach Anspruch 10, **dadurch gekennzeichnet, dass** die ermittelten Konzentrationen auf die Konzentration von Kreatinin in derselben Urinprobe normiert werden.

12. Verfahren nach Anspruch 10 oder 11, **dadurch gekennzeichnet, dass** die Urinprobe auf der Grundlage des berechneten Kennwerts in eine von zumindest zwei vordefinierten Gruppen eingeteilt wird.

13. Verfahren nach Anspruch 10 oder 11, **dadurch gekennzeichnet, dass** ein erweiterter Kennwert aus dem Kennwert errechnet wird, indem zusätzlich zumindest ein Wert aus der Gruppe berücksichtigt wird, die aus einem Wert, der die Kreatininkonzentration im Blutserum des Individuums angibt, einem Wert, der die glomeruläre Filtrationsrate des Individuums angibt, und einem Wert, der die geschätzte glomeruläre Filtrationsrate des Individuums angibt, besteht.

14. Verfahren nach Anspruch 13, **dadurch gekennzeichnet, dass** die Probe auf der Grundlage des berechneten erweiterten Kennwerts in eine von zumindest zwei vordefinierten Gruppen eingeteilt wird, wodurch ein Entscheidungshilfesystem bereitgestellt wird.

**Revendications**

1. Utilisation d'un ensemble de marqueurs comprenant au moins trois substances choisies parmi le groupe constitué de 3-hydroxy-isovalérate, acétyl carnitine, alanine, citrate, diméthylamine, glucose, glucuronate, hippurate, lactate, malonate, méthyl guanidine, malonate de méthyle, succinate de méthyle, p-crésol, acétate de phényle, phénylacétylglycine, phénylacétylglutamine, taurine, trigonelline et urée, dans un procédé in vitro pour déterminer le risque de rejet de rein d'un individu après avoir obtenu un don de rein par analyse d'urine, à condition que l'ensemble de marqueurs comprenne de l'alanine.

2. Utilisation selon la revendication 1, **caractérisée en ce que** l'ensemble de marqueurs comprend au moins une substance choisie parmi le groupe constitué de diméthylamine, glucuronate et urée.

3. Utilisation selon la revendication 1, **caractérisée en ce que** l'ensemble de marqueurs comprend de l'urée.

4. Utilisation selon l'une quelconque des revendications précédentes, **caractérisée en ce que** l'ensemble de marqueurs comprend du lactate et du citrate.

5. Utilisation selon l'une quelconque des revendications précédentes, **caractérisée en ce que** l'ensemble de marqueurs ne comprend pas de la diméthylamine, du lactate, de l'acétate et de l'alanine simultanément.

6. Utilisation selon l'une quelconque des revendications précédentes, **caractérisée en ce que** l'ensemble de marqueurs ne comprend pas seulement de la diméthylamine, du lactate et de l'alanine.

7. Utilisation selon l'une quelconque des revendications précédentes, **caractérisée en ce que** l'ensemble de marqueurs ne comprend pas seulement de la diméthylamine, de l'acétate et de l'alanine.

8. Utilisation selon l'une quelconque des revendications précédentes, **caractérisée en ce que** l'ensemble de marqueurs ne comprend pas seulement du lactate, de l'acétate et de l'alanine.

9. Ensemble de marqueurs comprenant au moins trois substances choisies parmi le groupe constitué de 3-hydroxy-isovalérate, acétyl carnitine, alanine, citrate, diméthylamine, glucose, glucuronate, hippurate, lactate, malonate, méthyl guanidine, malonate de méthyle, succinate de méthyle, p-crésol, acétate de phényle, phénylacétylglycine, phénylacétylglutamine, taurine, trigonelline et urée, destiné à être utilisé dans des diagnostics in vivo du risque de rejet de rein d'un individu après avoir reçu un don de rein par analyse d'urine, à condition que l'ensemble de marqueurs comprenne de l'alanine.

10. Procédé d'analyse in vitro d'un échantillon d'urine isolé, comprenant les étapes suivantes :

     a) détermination de la concentration d'au moins trois substances choisies parmi le groupe constitué de 3-hydroxy-isovalérate, acétyl carnitine, alanine, citrate, diméthylamine, glucose, glucuronate, hippurate, lactate, malonate, méthyl guanidine, malonate de méthyle, succinate de méthyle, p-crésol, acétate de phényle, phénylacétylglycine, phénylacétylglutamine, taurine, trigonelline et urée dans un échantillon d'urine provenant d'un individu qui a reçu un don de rein en analysant l'échantillon d'urine avec une technique de mesure adaptée, à condition que la concentration d'alanine soit déterminée, et
     b) calcul d'un score à partir des concentrations déterminées, le score étant indicatif du risque de rejet de rein de l'individu.

11. Procédé selon la revendication 10, **caractérisé en ce que** les concentrations déterminées sont normalisées sur la concentration de créatinine dans le même échantillon d'urine.

12. Procédé selon la revendication 10 ou 11, **caractérisé en ce que** l'échantillon d'urine est groupé dans un d'au moins deux groupes prédéfinis sur la base du score calculé.

13. Procédé selon la revendication 10 ou 11, **caractérisé en ce qu'**un score étendu est calculé à partir du score en considérant de manière supplémentaire au moins une valeur du groupe consistant en une valeur indiquant la concentration de créatinine dans le sérum sanguin de l'individu, une valeur indiquant le taux de filtration glomérulaire de l'individu, et une valeur indiquant le taux de filtration glomérulaire estimé de l'individu.

14. Procédé selon la revendication 13, **caractérisé en ce que** l'échantillon est groupé dans un d'au moins deux groupes prédéfinis sur la base du score étendu calculé, en fournissant ainsi un système d'aide à la décision.

FIG 1A

FIG 1B

FIG 1C

FIG 2A

FIG 2B

FIG 2C

FIG 2D

FIG 2E

AUC: 0.830 (0.763–0.897)

FIG 2F

AUC: 0.836 (0.771–0.901)

FIG 2G

FIG 3A

FIG 3B

FIG 3C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012045773 A9
- US 9541620 B2
- WO 2002010755 A2
- US 20080161228 A1

### Non-patent literature cited in the description

- **MALYSZKO ; JOLANTA et al.** Biomarkers of delayed graft function as a form of acute kidney injury in kidney transplantation. *Scientific Reports,* 2015, vol. 5, 11684
- **SUHRE, K. et al.** Urine metabolite profiles predictive of human kidney allograft status. *Journal of the American Society of Nephrology,* 2016, vol. 27 (2), 626-636